# EUROPEAN PATENT APPLICATION

(11) **EP 2 233 589 A1**
(43) Date of publication of application: **29.09.2010**
(21) Application number: 09305248.8
(22) Date of filing: 19.03.2009
(51) Int. Cl.: C12Q 1/68

(54) **MSH2 and adjuvant cisplatin-based chemotherapy in non-small-cell lung cancer**

(71) Applicant: Institut Gustave Roussy, 94800 Villejuif (FR)
(72) Inventor: Fouret, Pierre, 75116 Paris (FR); Soria, Jean-Charles, 91430 Igny (FR)
(74) Representative: Warcoin, Jacques

(57) **Abstract**

The invention is generally directed to a diagnostic method for predicting the benefit of the response of a subject diagnosed with cancer to a platinum compound-based adjuvant chemotherapy, preferably to a cisplatin-based chemotherapy which implements the determination of the MSH2 expression level in the biological sample containing tumor cells, and, optionally, the ERCC1 expression level.

SEQUENCE LISTING

<110>  IGR (Institut Gustave-Roussy)

<120>  MSH2 AND ADJUVANT CISPLATIN-BASED CHEMOTHERAPY IN NON-SMALL-CELL
       LUNG CANCER

<130>  67833/D27163

<160>  9

<170>  PatentIn version 3.3

<210>  1
<211>  934
<212>  PRT
<213>  Homo sapiens


<220>
<221>  misc_feature
<223>  DNA mismatch repair protein Msh2 (MutS protein homolog 2)
       (UniProtKB: P43246)

<400>  1

<210>  2
<211>  323
<212>  PRT
<213>  Homo sapiens

<220>
<221>  misc_feature
<223>  Excision repair cross-complementing 1 isoform 1 (GenBank
       NP_973730).

<400>  2

Met Asp Pro Gly Lys Asp Lys Glu Gly Val Pro Gln Pro Ser Gly Pro
1               5                   10                  15

Pro Ala Arg Lys Lys Phe Val Ile Pro Leu Asp Glu Asp Glu Val Pro
            20                  25                  30

Pro Gly Val Ala Lys Pro Leu Phe Arg Ser Thr Gln Ser Leu Pro Thr
        35                  40                  45

Val Asp Thr Ser Ala Gln Ala Ala Pro Gln Thr Tyr Ala Glu Tyr Ala
    50                  55                  60

Ile Ser Gln Pro Leu Glu Gly Ala Gly Ala Thr Cys Pro Thr Gly Ser
65                  70                  75                  80

Glu Pro Leu Ala Gly Glu Thr Pro Asn Gln Ala Leu Lys Pro Gly Ala
                85                  90                  95

Lys Ser Asn Ser Ile Ile Val Ser Pro Arg Gln Arg Gly Asn Pro Val
            100                 105                 110

Leu Lys Phe Val Arg Asn Val Pro Trp Glu Phe Gly Asp Val Ile Pro
        115                 120                 125

Asp Tyr Val Leu Gly Gln Ser Thr Cys Ala Leu Phe Leu Ser Leu Arg
    130                 135                 140

Tyr His Asn Leu His Pro Asp Tyr Ile His Gly Arg Leu Gln Ser Leu
145                 150                 155                 160

Gly Lys Asn Phe Ala Leu Arg Val Leu Leu Val Gln Val Asp Val Lys
                165                 170                 175

Asp Pro Gln Gln Ala Leu Lys Glu Leu Ala Lys Met Cys Ile Leu Ala
            180                 185                 190

Asp Cys Thr Leu Ile Leu Ala Trp Ser Pro Glu Glu Ala Gly Arg Tyr
        195                 200                 205

Leu Glu Thr Tyr Lys Ala Tyr Glu Gln Lys Pro Ala Asp Leu Leu Met
    210                 215                 220

Glu Lys Leu Glu Gln Asp Phe Val Ser Arg Val Thr Glu Cys Leu Thr
225                 230                 235                 240

Thr Val Lys Ser Val Asn Lys Thr Asp Ser Gln Thr Leu Leu Thr Thr
                245                 250                 255

Phe Gly Ser Leu Glu Gln Leu Ile Ala Ala Ser Arg Glu Asp Leu Ala
            260                 265                 270

Leu Cys Pro Gly Leu Gly Pro Gln Lys Val Arg Ala Leu Gly Lys Asn
        275                 280                 285

Pro Arg Ser Trp Gly Lys Glu Arg Ala Pro Asn Lys His Asn Leu Arg
    290                 295                 300

Pro Gln Ser Phe Lys Val Lys Lys Glu Pro Lys Thr Arg His Ser Gly
305                 310                 315                 320

Phe Arg Leu

<210>  3
<211>  297
<212>  PRT
<213>  Homo sapiens

<220>

<221>  misc_feature
<223>  Excision repair cross-complementing 1 isoform 2 (UniProtKB:
      P07992).

<400>  3

Met Asp Pro Gly Lys Asp Lys Glu Gly Val Pro Gln Pro Ser Gly Pro
1               5                   10                  15

Pro Ala Arg Lys Lys Phe Val Ile Pro Leu Asp Glu Asp Glu Val Pro
            20                  25                  30

Pro Gly Val Ala Lys Pro Leu Phe Arg Ser Thr Gln Ser Leu Pro Thr
        35                  40                  45

Val Asp Thr Ser Ala Gln Ala Ala Pro Gln Thr Tyr Ala Glu Tyr Ala
    50                  55                  60

Ile Ser Gln Pro Leu Glu Gly Ala Gly Ala Thr Cys Pro Thr Gly Ser
65                  70                  75                  80

Glu Pro Leu Ala Gly Glu Thr Pro Asn Gln Ala Leu Lys Pro Gly Ala
                85                  90                  95

Lys Ser Asn Ser Ile Ile Val Ser Pro Arg Gln Arg Gly Asn Pro Val
            100                 105                 110

Leu Lys Phe Val Arg Asn Val Pro Trp Glu Phe Gly Asp Val Ile Pro
        115                 120                 125

Asp Tyr Val Leu Gly Gln Ser Thr Cys Ala Leu Phe Leu Ser Leu Arg
    130                 135                 140

Tyr His Asn Leu His Pro Asp Tyr Ile His Gly Arg Leu Gln Ser Leu
145                 150                 155                 160

Gly Lys Asn Phe Ala Leu Arg Val Leu Leu Val Gln Val Asp Val Lys
                165                 170                 175

Asp Pro Gln Gln Ala Leu Lys Glu Leu Ala Lys Met Cys Ile Leu Ala
            180                 185                 190

Asp Cys Thr Leu Ile Leu Ala Trp Ser Pro Glu Glu Ala Gly Arg Tyr
        195                 200                 205

Leu Glu Thr Tyr Lys Ala Tyr Glu Gln Lys Pro Ala Asp Leu Leu Met
    210                 215                 220

Glu Lys Leu Glu Gln Asp Phe Val Ser Arg Val Thr Glu Cys Leu Thr
225                 230                 235                 240

Thr Val Lys Ser Val Asn Lys Thr Asp Ser Gln Thr Leu Leu Thr Thr
                245                 250                 255

Phe Gly Ser Leu Glu Gln Leu Ile Ala Ala Ser Arg Glu Asp Leu Ala
            260                 265                 270

Leu Cys Pro Gly Leu Gly Pro Gln Lys Ala Arg Arg Leu Phe Asp Val
        275                 280                 285

Leu His Glu Pro Phe Leu Lys Val Pro
    290                 295

<210>  4
<211>  3145
<212>  DNA
<213>  Homo sapiens

<220>
<221>  misc_feature
<223>  mRNA encoding DNA mismatch repair protein Msh2 (MutS protein
       homolog 2) (GenBank; NM_000251)

<400>  4
ggcgggaaac agcttagtgg gtgtggggtc gcgcattttc ttcaaccagg aggtgaggag      60

gtttcgacat ggcggtgcag ccgaaggaga cgctgcagtt ggagagcgcg gccgaggtcg     120

gcttcgtgcg cttctttcag ggcatgccgg agaagccgac caccacagtg cgccttttcg     180

accggggcga cttctatacg gcgcacggcg aggacgcgct gctggccgcc cgggaggtgt     240

tcaagaccca gggggtgatc aagtacatgg ggccggcagg agcaaagaat ctgcagagtg     300

ttgtgcttag taaaatgaat tttgaatctt ttgtaaaaga tcttcttctg gttcgtcagt     360

atagagttga agtttataag aatagagctg gaaataaggc atccaaggag aatgattggt     420

atttggcata taaggcttct cctggcaatc tctctcagtt tgaagacatt ctctttggta     480

acaatgatat gtcagcttcc attggtgttg tgggtgttaa aatgtccgca gttgatggcc     540

agagacaggt tggagttggg tatgtggatt ccatacagag gaaactagga ctgtgtgaat     600

tccctgataa tgatcagttc tccaatcttg aggctctcct catccagatt ggaccaaagg     660

aatgtgtttt acccggagga gagactgctg gagacatggg gaaactgaga cagataattc     720

aaagaggagg aattctgatc acagaaagaa aaaaagctga cttttccaca aaagacattt     780

atcaggacct caaccggttg ttgaaaggca aaaagggaga gcagatgaat agtgctgtat     840

tgccagaaat ggagaatcag gttgcagttt catcactgtc tgcggtaatc aagtttttag 900

aactcttatc agatgattcc aactttggac agtttgaact gactactttt gacttcagcc 960

agtatatgaa attggatatt gcagcagtca gagcccttaa cctttttcag ggttctgttg 1020

aagataccac tggctctcag tctctggctg ccttgctgaa taagtgtaaa acccctcaag 1080

gacaaagact tgttaaccag tggattaagc agcctctcat ggataagaac agaatagagg 1140

agagattgaa tttagtggaa gcttttgtag aagatgcaga attgaggcag actttacaag 1200

aagatttact tcgtcgattc ccagatctta accgacttgc caagaagttt caaagacaag 1260

cagcaaactt acaagattgt taccgactct atcagggtat aaatcaacta cctaatgtta 1320

tacaggctct ggaaaaacat gaaggaaaac accagaaatt attgttggca gtttttgtga 1380

ctcctcttac tgatcttcgt tctgacttct ccaagtttca ggaaatgata gaaacaactt 1440

tagatatgga tcaggtggaa aaccatgaat tccttgtaaa accttcattt gatcctaatc 1500

tcagtgaatt aagagaaata atgaatgact tggaaaagaa gatgcagtca acattaataa 1560

gtgcagccag agatcttggc ttggaccctg gcaaacagat taaactggat tccagtgcac 1620

agtttggata ttactttcgt gtaacctgta aggaagaaaa agtccttcgt aacaataaaa 1680

actttagtac tgtagatatc cagaagaatg gtgttaaatt taccaacagc aaattgactt 1740

ctttaaatga agagtatacc aaaaataaaa cagaatatga agaagcccag gatgccattg 1800

ttaaagaaat tgtcaatatt tcttcaggct atgtagaacc aatgcagaca ctcaatgatg 1860

tgttagctca gctagatgct gttgtcagct ttgctcacgt gtcaaatgga gcacctgttc 1920

catatgtacg accagccatt ttggagaaag gacaaggaag aattatatta aaagcatcca 1980

ggcatgcttg tgttgaagtt caagatgaaa ttgcatttat tcctaatgac gtatactttg 2040

aaaaagataa acagatgttc cacatcatta ctggccccaa tatgggaggt aaatcaacat 2100

atattcgaca aactggggtg atagtactca tggcccaaat tgggtgtttt gtgccatgtg 2160

agtcagcaga agtgtccatt gtggactgca tcttagcccg agtaggggct ggtgacagtc 2220

aattgaaagg agtctccacg ttcatggctg aaatgttgga aactgcttct atcctcaggt 2280

ctgcaaccaa agattcatta ataatcatag atgaattggg aagaggaact tctacctacg 2340

atggatttgg gttagcatgg gctatatcag aatacattgc aacaaagatt ggtgcttttt 2400

gcatgtttgc aacccatttt catgaactta ctgccttggc caatcagata ccaactgtta 2460

ataatctaca tgtcacagca ctcaccactg aagagacctt aactatgctt tatcaggtga 2520

agaaaggtgt ctgtgatcaa agttttggga ttcatgttgc agagcttgct aatttcccta 2580

agcatgtaat agagtgtgct aaacagaaag ccctggaact tgaggagttt cagtatattg 2640

gagaatcgca aggatatgat atcatggaac cagcagcaaa gaagtgctat ctggaaagag 2700

agcaaggtga aaaaattatt caggagttcc tgtccaaggt gaaacaaatg ccctttactg 2760

aaatgtcaga agaaaacatc acaataaagt taaaacagct aaaagctgaa gtaatagcaa 2820

agaataatag ctttgtaaat gaaatcattt cacgaataaa agttactacg tgaaaaatcc 2880

cagtaatgga atgaaggtaa tattgataag ctattgtctg taatagtttt atattgtttt 2940

atattaaccc tttttccata gtgttaactg tcagtgccca tgggctatca acttaataag 3000

atatttagta atattttact ttgaggacat tttcaaagat ttttattttg aaaaatgaga 3060

gctgtaactg aggactgttt gcaattgaca taggcaataa taagtgatgt gctgaatttt 3120

ataaataaaa tcatgtagtt tgtgg 3145

<210> 5
<211> 1273
<212> DNA
<213> Homo sapiens

<220>
<221> misc_feature
<223> transcript variant 1, mRNA (GenBank NM_202001) encoding the
 excision repair cross-complementing 1 isoform 1 protein

<400> 5
aaaggggcgt ggcgttacag agcctctagc gctgggtgtt ggggacctga cgctatggag 60

ctctcggagt tttgtggggg acggctgtga gtggggggtt cctgctgcgg gatgagaacg 120

tagacgccag tggctcactc gctcctggca ccttcccttt caggctccag atggaccctg 180

ggaaggacaa agagggggtg ccccagccct cagggccgcc agcaaggaag aaatttgtga 240

tacccctcga cgaggatgag gtccctcctg gagtggccaa gcccttattc cgatctacac 300

agagccttcc cactgtggac acctcggccc aggcggcccc tcagacctac gccgaatatg 360

ccatctcaca gcctctggaa ggggctgggg ccacgtgccc cacagggtca gagcccctgg 420

caggagagac gcccaaccag gccctgaaac ccggggcaaa atccaacagc atcattgtga 480

gccctcggca gaggggcaat cccgtactga agttcgtgcg caatgtgccc tgggaatttg 540

gcgacgtaat tcccgactat gtgctgggcc agagcacctg tgccctgttc ctcagcctcc 600

gctaccacaa cctgcaccca gactacatcc atgggcggct gcagagcctg gggaagaact 660

tcgccttgcg ggtcctgctt gtccaggtgg atgtgaaaga tccccagcag gccctcaagg 720

agctggctaa gatgtgtatc ctggccgact gcacattgat cctcgcctgg agccccgagg 780

aagctgggcg gtacctggag acctacaagg cctatgagca gaaaccagcg gacctcctga 840

tggagaagct agagcaggac ttcgtctccc gggtgactga atgtctgacc accgtgaagt 900

cagtcaacaa aacggacagt cagaccctcc tgaccacatt tggatctctg gaacagctca 960

tcgccgcatc aagagaagat ctggccttat gcccaggcct gggccctcag aaagtaagag 1020

ctctgggaaa gaacccaagg agttggggga aggagagagc cccaaataaa cacaacctga 1080

gaccccaaag ttttaaggtg aaaaaagaac caaagaccag acacagtggc ttccgcctgt 1140

aatcccaaca ttttgggagg ccaaggcggg aggactgctt gaggccagaa gttggagacc 1200

agcctgggca agtggacacc tcatttttac taaaaataaa aaaaactagc tgggcaaaaa 1260

aaaaaaaaaa aaa 1273

<210> 6
<211> 1101
<212> DNA
<213> Homo sapiens

<220>
<221> misc_feature
<223> transcript variant 2, mRNA (GenBank NM_001983) encoding the
excision repair cross-complementing 1 isoform 2 protein

<400> 6
ccggaagtgc tgcgagccct gggccacgct ggccgtgctg gcagtgggcc gcctcgatcc 60

ctctgcagtc tttcccttga ggctccaaga ccagcaggtg aggcctcgcg gcgctgaaac 120

cgtgaggccc ggaccacagg ctccagatgg accctgggaa ggacaaagag ggggtgcccc 180

agccctcagg gccgccagca aggaagaaat ttgtgatacc cctcgacgag gatgaggtcc 240

ctcctggagt ggccaagccc ttattccgat ctacacagag ccttcccact gtggacacct 300

cggcccaggc ggcccctcag acctacgccg aatatgccat ctcacagcct ctggaagggg 360

ctggggccac gtgccccaca gggtcagagc ccctggcagg agagacgccc aaccaggccc 420

tgaaacccgg ggcaaaatcc aacagcatca ttgtgagccc tcggcagagg ggcaatcccg 480

tactgaagtt cgtgcgcaat gtgccctggg aatttggcga cgtaattccc gactatgtgc 540

tgggccagag cacctgtgcc ctgttcctca gcctccgcta ccacaacctg cacccagact 600

acatccatgg gcggctgcag agcctgggga agaacttcgc cttgcgggtc ctgcttgtcc 660

aggtggatgt gaaagatccc cagcaggccc tcaaggagct ggctaagatg tgtatcctgg 720

ccgactgcac attgatcctc gcctggagcc ccgaggaagc tgggcggtac ctggagacct 780

acaaggccta tgagcagaaa ccagcggacc tcctgatgga gaagctagag caggacttcg 840

tctcccgggt gactgaatgt ctgaccaccg tgaagtcagt caacaaaacg gacagtcaga 900

ccctcctgac cacatttgga tctctggaac agctcatcgc cgcatcaaga gaagatctgg 960

ccttatgccc aggcctgggc cctcagaaag cccggaggct gtttgatgtc ctgcacgagc 1020

ccttcttgaa agtaccctga tgaccccagc tgccaaggaa acccccagtg taataataaa 1080

tcgtcctccc aggccaggct c                                              1101

<210>  7
<211>  21
<212>  DNA
<213>  Artificial sequence

<220>
<223>  Forward primer for amplification of mRNA fragment encoding human
       ERCC1

<400>  7
gggaatttgg cgacgtaatt c                                                21

<210>  8
<211>  18
<212>  DNA
<213>  Artificial sequence

<220>
<223>  Reward primer for amplification of mRNA fragment encoding human
       ERCC1

<400>  8
gcggaggctg aggaacag                                                    18

<210>  9
<211>  25
<212>  DNA
<213>  Artificial sequence

<220>
<223>  6FAM/TAMRA labeled probe for quantitative PCR of mRNA encoding
       human ERCC1

<400>  9
cacaggtgct ctggcccagc acata                                            25

## Description

The invention is generally directed to a diagnostic method for predicting the benefit of the response of a subject diagnosed with cancer to a platinum compound-based adjuvant chemotherapy, preferably to a cisplatin-based chemotherapy which implements the determination of the MSH2 expression level in the biological sample containing tumor cells, and, optionally, the ERCC 1 expression level.

Platinum compound-based adjuvant chemotherapy, such as cisplatin, has become a new standard of care for cancer patients, particulary non-small cell lung cancer (NSCLC) patients. Platinum compounds are a hallmark of chemotherapy against lung cancer due to their DNA binding capacity which results in DNA damage and cell death. As DNA repair has a determinant role in both cancer susceptibility (high DNA repair capacity is a barrier against carcinogenesis) and drug resistance (high DNA repair capacity prevents platinum-induced DNA damage), it has been suggested to play a Janus-faced role in cancer physiology¹.

*MutS homolog 2 (MSH2),* which is frequently mutated in hereditary nonpolyposis colon cancer, encodes a component of the mismatch repair pathway ². MSH2 binds to DNA mismatches, thereby initiating DNA repair. MSH2 also binds to cisplatin-induced DNA cross-links, thereby initiating their excision and repair ^{3, 4}. The loss of MSH2 may therefore impair the repair of cisplatin-induced DNA cross-links, which are highly deleterious to tumour cells. Indeed, MSH2 is required, together with critical components of other DNA damage repair pathways, such as the excision repair cross-complementing group 1 (ERCC1) protein, to repair cisplatin-induced DNA interstrand cross-links ^{5,6}_{.}

MSH2 expression is reduced in 10 % to 58 % of NSCLC ⁷⁻¹⁶. A recent study reported that loss of MSH2 expression in tumours from patients with advanced NSCLC led to higher rates of response to oxaliplatin-based chemotherapy, but not to cisplatin-based chemotherapy¹⁴. In a larger study of genetic polymorphisms among patients with advanced NSCLC, the MSH2 gIV12-6T>C variant was associated with low MSH2 expression and better response to cisplatin.

The biology of the tumor is of a great interest when choosing the optimal therapy for patients with cancer, particularly for NSCLC. A number of potential biomarkers is under investigation in the hope that it will be possible to identify markers that assist in the selection of patients for specific therapies.

Promising results so far suggest that customized therapy for individual patients with the help of predictive biomarkers is possible and it is likely that this strategy will improve treatment of NSCLC in the future.

Thus, it remains desirable to provide new marker capable to predict the benefit of the response of a subject diagnosed with cancer, particularly with NSCLC, to a platinum-based chemotherapy such as cisplatin-based chemotherapy.

This is the object of the present invention.

In a first aspect, the invention is directed to a method for *in vitro* predicting the benefit of the response of a subject diagnosed with cancer to a platinum-based chemotherapy from a biological sample from said subject comprising the following steps of:
a) determining the MSH2 expression level in the biological sample; and
b) optionally, comparing said MSH2 expression level to the MSH2 expression level of a reference control or population, or compared to the non tumor cells content.
   In a preferred embodiment, the method according to the present invention further comprises the following steps of:
c) determining the ERCC1 expression level from the same or from another biological sample; and
d) optionally, comparing said ERCC1 expression level to the ERCC1 expression level of a reference control or population, or compared to the non tumor cells content.

According to the present invention, if the MSH2 expression level obtained for the patient biological sample is less or equal than the expression level obtained for said reference population, or is decreased compared to the non tumor cells, then an overall survival benefit can be predicted.

According to the present invention, if the MSH2 and the ERCC1 expression levels obtained for the patient biological sample are less or equal than the expression level obtained for said reference populations, or is decreased compared to the non tumor cells then an overall survival benefit can be predicted.

In a second aspect, the invention is directed to a method for in *vitro* assessing whether a platinum-based chemotherapy is appropriate for a subject diagnosed with cancer from a biological sample from said subject, said method comprising the following steps of:
a) determining the MSH2, and optionally the ERCC1, expression level in said biological sample; and
b) optionally, comparing said MSH2, and optionally ERCC1, expression level to the MSH2, and optionally ERCC1, of a reference control or population, or compared to the non tumor cells content,
   - a platinum-based chemotherapy will be determined as an appropriate chemotherapy if the MSH2, and optionally the ERCC1, expression level is less or equal than the MSH2, and optionally the ERCC1, expression level of a reference control or population, or is decreased compared to the non tumor cells, and
   - a platinum-free chemotherapy will be determined as an appropriate chemotherapy if the MSH2, and optionally the ERCC1, expression level is greater than the MSH2, and optionally the ERCC1, expression level of said reference control or population, or is increased compared to the non tumor cells,.

In a third aspect, the invention is directed to a method for *in vitro* selecting a subject diagnosed from cancer for a treatment with a platinum-based chemotherapy from a biological sample from said subject, said method comprising the steps of:
a) determining the MSH2, and optionally the ERCC1, expression level in said biological sample; and
b) optionally, comparing said MSH2, and optionally ERCC1, expression level to the MSH2, and optionally ERCC1, of a reference control or population, or compared to the non tumor cells content
wherein said subject will be selected for a platinum-based chemotherapy if the MSH2, and optionally the ERCC1, expression level is less or equal than the MSH2, and optionally the ERCC1, expression level of a reference control or population, or is decreased compared to the non tumor cells.

Preferably, said subject is a human patient.

In a preferred embodiment, said chemotherapy is an adjuvant-platinum-based chemotherapy.

In a more preferred embodiment, said platinum-based chemotherapy or said adjuvant-platinum-based chemotherapy is selected from the group consisting of cisplatin-, carboplatin- or oxaliplatin-based chemotherapy, the most preferred being cisplatin-based chemotherapy.

In another aspect, the present invention comprises the use of MSH2 as a prognostic marker associated with longer overall survival of a subject suffering from cancer.

In the method of the present invention or for the use of MSH2 as a prognostic marker of the invention, the diagnosed cancer or the subject suffers from a cancer selected from the group of cancer consisting of malignant mesothelioma, bladder cancer, testicular cancer, cancer of the upper aero-digestive tract or ovarian cancer.

Preferably, these patients suffering from this cancer are not patients presenting the Lynch syndrome (also called hereditary nonpolyposis colorectal cancer) which is one the most common hereditary colorectal cancer syndrome and often associated with mutations in the MSH2 gene or in the MLH1 gene (also related to the mismatch repair system).

In a preferred embodiment of the method of the present invention or for the use of MSH2 as a prognostic marker of the invention, the diagnosed cancer is the NSCLC.

In a preferred embodiment of the method of the present invention said biological sample from the patient suffered from a cancer is a tissue sample comprising cancer cells, particularly a biopsy including tissue containing tumor cells.

Said tissue sample can be fixed, paraffin-embedded, or fresh, or frozen.

In a preferred embodiment of the method of the present invention in step a) and/or in step c), said MSH2 expression and/or said ERCC1 expression level(s) to be determined is (are) the level of the RNA transcript or their expression protein product of the MSH2 and/or ERCC1 gene.

In a preferred embodiment the MSH2 and/or ERCC1 expression level is determined by:
- a method including a PCR or a RT-PCR method, or a Northern blot method when the determined MSH2 and/or ERCC1 expression product is the RNA transcript; or
- a Western blot method or an immunohistochemistry method when the determined MSH2 and/or ERCC1 expression product is the MSH2 and/or ERCC1 protein, or a specific fragment thereof.

In a preferred embodiment, in the step of determining the MSH2 and/or the ERCC1 expression level in the biological sample, the MSH2 and/or the ERCC1 expression products are the MSH2 and/or the ERCC1 protein, or a specific fragment thereof.

In a more preferred embodiment, in the step of determining the MSH2 and/or the ERCC1 expression level in the biological sample, preferably in tissue sample from the tumor, the MSH2 and/or the ERCC1 expression protein, is determined by immunohistochemistry.

Preferably, when using an immunohistochemistry method on a tumor tissue sample from the subject, the MSH2 nuclear and, optionally, the ERCC1 nuclear reactivity is determined. Preferably reactive lung or stroma cells can be served as internal positive control and the tumour cells can be graded using the expression level in fibroblasts or endothelial cells as reference.

In a preferred embodiment, ERCC1-specific immunostaining is carried out as depicted in Olaussen et al. 2006 (18), see also Olaussen et al., 2007 (New England Journal of Medecine, 2007, ERCC1-Specific Immunostaining in Non-Small-Cell Lung Cancer.Volume 357, Number 15:1559-1561).

In a preferred embodiment, the specific anti MSH2 and, optionally, the anti-ERCC1antibody used for the immunohistochemistry method, or comprised into the kit of the present invention, are directed specifically against the MSH2 or the ERCC1 human protein selected from the group consisting of the proteins having the following reference sequences:
- DNA mismatch repair protein MSH2 (MutS protein homolog 2) (UniProtKB: P43246, SEQ ID N° 1);
- Excision repair cross-complementing 1 isoform 1 (GenBank NP_973730, SEQ ID N° 2); and
- Excision repair cross-complementing 1 isoform 2 (UniProtKB: P07992, SEQ ID N° 3).

In a more preferred embodiment, for ERCC1, the kit of the present invention or the step of immunostaining or immunohistochemistry analysis in the method of the present invention is carried out using anti-ERCC1 monoclonal or polyclonal antibody, monoclonal being the most preferred, obtained by using the full length ERCC1 protein, isoform 1 or 2, as antigen for immunization.

In an also more preferred embodiment, for ERCC1, said kit or immunostaining or immunohistochemistry analysis is carried out using anti-ERCC1 antibody directed against a common epitope of the isoform 1 and 2 sequence (located in the consensus protein domain), the resulting antibody being able to recognize the ERCC1 isoform 1 and 2 protein.

In the most preferred embodiment, the kit of the present invention or the step of ERCC1-specific immunostaining of the method of the present invention is carried out by using a monoclonal antibody specifically directed against the full length recombinant human ERCC1 protein isoform 2.

In an also preferred embodiment, in the step of determining the MSH2 and/or the ERCC1 expression level in the biological sample, the MSH2 and/or the ERCC1 expression products are the MSH2 and/or the ERCC1 mRNAs, or a specific fragment thereof.

Thus, the determination of the MSH2 and/or the ERCC1 mRNA can be carried out by a method which comprises the following steps:
A) extraction of the total RNAs of said biological sample, preferably from tumor cells, followed, where appropriate, by purification of the mRNAs;
B) reverse transcription of the RNAs extracted in step A) via an oligo dT primer; and
C) PCR amplification of the cDNAs obtained in step B) using at least a pair of primers specific for the MSH2 and/or the ERCC1 mRNA to be quantified.

In a preferred embodiment, when the determination of the MSH2 and/or the ERCC1 mRNA is carried out by a method comprising a PCR or RT-PCR amplification or in the kit of the present invention, primers and probe set specific for the MSH2 and/or the ERCC1 mRNA to be quantified. can be designed using the following reference sequences:
- mRNA encoding DNA mismatch repair protein MSH2 (MutS protein homolog 2) (GenBank; NM_000251; SEQ ID N° 4);
- transcript variant 1, mRNA (GenBank NM_202001; SEQ ID N° 5) encoding the excision repair cross-complementing 1 isoform 1 protein ERCC1; and
- transcript variant 2, mRNA (GenBank NM_001983, SEQ ID N° 6) encoding the excision repair cross-complementing 1 isoform 2 protein ERCC1.

In a more preferred embodiment, for ERCC1 mRNA expression analysis by RT-QPCR, or in the kit of the present invention, the primer and probe sets can be designed in order to result to the quantification of the presence of an amplicon which is common to the transcript variant 1 and 2 sequence (located in the consensus domain, preferably, the primers having SEQ ID NOs/ 7 and 8, and, optionally, the probe having the sequence SEQ ID NO/ 9).

In the most preferred embodiment, for ERCC1 mRNA expression analysis by RT-QPCR, or in the kit of the present invention, the primer and probe sets can be designed in order to result to the quantification of the presence of a fragment amplicon comprised in the mRNA sequence encoding the full length recombinant human ERCC1 protein isoform 2.

In another aspect, the present invention is directed to a kit or an array, preferably for *in vitro* predicting the benefit of the response of a subject diagnosed with cancer to a platinum-based chemotherapy, wherein said kit or array comprises a reagent for assaying MSH2 expression and a reagent for assaying ERCC1 expression in a biological sample from a patient, and, optionally, an instruction sheet.

In a preferred embodiment, in the kit or array according to the present invention, the reagent for assaying MSH2 and the reagent for assaying ERCC1 expression comprises a reagent selected from the group consisting of:
- a probe or a pair of primers that hybridizes specifically to the MSH2 or to the ERCC1 mRNA or cDNA; or
- an anti-MSH2 or an anti-ERCC1 antibody for performing a Western blot or immunohistochemistry assay.

Western blotting or immunohistochemistry method can be used for analysing or quantifying specific protein expression in biological sample. Such methods are well known from the skilled man.

The blots can be detected using antibodies specifically directed against different specific regions or epitope of MSH2 or ERCC1 protein, particularly against human MSH2 or ERCC1 protein.

The term "antibody" as used herein refers to immunoglobulin molecules and immunologically active portions of immunoglobulin molecules, i.e., molecules that contain an antigen binding site which specifically binds (immunoreacts with) the MSH2 or the ERCC1 protein. The term "antibody" comprises monoclonal or polyclonal antibodies but also chimeric or humanized antibodies.

An isolated MSH2 or ERCC1 protein, such as recombinant protein, or a specific fragment thereof can be used as an immunogen to generate antibodies that bind such protein using standard techniques for polyclonal and monoclonal antibody preparation. It may be also possible to use any fragment of these protein which contains at least one antigenic determinant may be used to generate these specific antibodies.

A protein immunogen typically is used to prepare antibodies by immunizing a suitable subject, (e.g., rabbit, goat, mouse or other mammal) with the immunogen. An appropriate immunogenic preparation can contain said protein, or fragment thereof, and further can include an adjuvant, such as Freund's complete or incomplete adjuvant, or similar immunostimulatory agent.

Thus, antibody for use in accordance with the invention include either polyclonal, monoclonal chimeric or humanized antibodies able to selectively bind, or which selectively bind to an epitope-containing a polypeptide comprising a contiguous span of at least 8 to 10 amino acids of an amino acid sequence of the MSH2 or the ERCC1 protein, preferably the human protein.

In a preferred embodiment, quantitative RT-PCR analysis can be used to measure the mRNA expression levels of MSH2 and ERCC1 in a biological sample or tissue sample. Gene expression analysis by real-time quantitative PCR (RT-QPCR) is well known from the skilled person.

For example MSH2 and ERCC1 mRNA expression analysis by RT-QPCR can be assessed after standard tissue sample RNA extraction (for example the samples are lysed in a tris-chloride, EDTA, sodium dodecyl sulphate and proteinase K containing buffer and RNA is then extracted with phenol-chloroform-isoamyl alcohol followed by precipitation with isopropanol in the presence of glycogen and sodium acetate). RNA is then resuspended in diethyl pyrocarbonate water (Ambion Inc., Austin, TX) and treated with DNAse I (Ambion Inc., Austin, TX) to avoid DNA contamination. Complementary DNA was synthesized using for example Maloney Murine Leukemia Virus retrotranscriptase enzyme. Template cDNA was added to Taqman Universal Master Mix (AB, Applied Biosystems, Foster City, CA) in a 12.5-µl reaction with specific primers and probe for each gene. The primer and probe sets can be designed using Primer Express 2.0 Software (AB) and the reference sequences (which can be obtained on the web site http://www.ncbi.nlm.nih.gov/entrez/query.fcgi?db=gene). Primers and probe for the ERCC1 mRNA expression analysis by RT-QPCR can be:
- ERCC1 (reference Genbank sequence NM_001983 3-4, Exon Boundary 3-4) (Bellmunt et al., Annals of Oncology 2007 18(3):522-528).

Forward primer 5' GGG AAT TTG GCG ACG TAA TTC 3' (SEQ ID NO : 7);

Reward primer 5' GCG GAG GCT GAG GAA CAG 3' 3' (SEQ ID NO : 8); and

Labeled Probe 6FAM 5' CAC AGG TGC TCT GGC CCA GCA CAT A 3' TAMRA (SEQ ID NO : 9).
MSH2 : labeled probe-mix from Applied Biosystems can be used (Helleman et al, BMC Cancer. 2006; 6: 201);
- Applied Biosystems, TaqMan® Gene Expression Assays, Assay ID Hs00179887_ml (Reference Sequence: GenBank NM_000251.1, Translated protein: NP_000242.1 Exon Boundary: 14-15; location 2529; Amplicon Length: 87).

Quantification of gene expression was carried out using the ABI Prism 7900HT Sequence Detection System (AB).

A preferred agent for detecting and quantifying mRNA or cDNA encoding the MSH2 or the ERCC1 protein, is a labeled nucleic acid probe or primers able to hybridize this mRNA or cDNA. The nucleic acid probe can be an oligonucleotide of at least 10, 15, 30, 50 or 100 nucleotides in length and sufficient to specifically hybridize under stringent conditions to the mRNA or cDNA. The nucleic acid primer can be an oligonucleotide of at least 10, 15 or 20 nucleotides in length and sufficient to specifically hybridize under stringent conditions to the mRNA or cDNA, or complementary sequence thereof (preferred are oligonucleotide primers or probe having at least 90 %, 95 %, 99 % and 100 % identity with the mRNA sequence fragment or the complementary sequence thereof) .

A preferred agent for detecting and quantifying the MSH2 or the ERCC1 protein, is an antibody able to bind specifically to this protein, preferably an antibody with a detectable label. Antibodies can be polyclonal, or more preferably, monoclonal. An intact antibody, or a fragment thereof (e.g., Fab or F(ab')2) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin.

For example, *in vitro* techniques for detection of candidate mRNA include Northern hybridizations and in situ hybridizations. *In vitro* techniques for detection of the candidate protein include enzyme linked immunosorbent assays (ELISAs), Western blots, immunoprecipitations and immunofluorescence. *In vitro* techniques for detection of candidate cDNA include Southern hybridizations.

When the invention encompasses kits for quantifying the level of the MSH2 and the ERCC1 protein, the kit can comprise a labeled compound or agent capable of quantifying these proteins. Said agents can be packaged in a suitable container. The kit can further comprise instructions for using the kit to quantify the level of the MSH2 and the ERCC1 protein, or of the MSH2 and the ERCC1 transcript.

In certain embodiments of the method of the present invention, the determination of the MSH2 and the ERCC1 transcripts involves the use of a probe/primer in a polymerase chain reaction (PCR), such as anchor PCR or RACE PCR, or, alternatively, in a ligation chain reaction (LCR), or alternatively quantitative real time RT-PCR. This method can include the steps of collecting a sample of tumor cells from a patient, isolating nucleic acid (e.g. mRNA) from the tumor cells of the sample, optionally transforming mRNA into corresponding cDNA, contacting the nucleic acid sample with one or more primers which specifically hybridize to the MSH2 or the ERCC1 mRNA or their corresponding cDNA under conditions such that hybridization and amplification of the MSH2 or the ERCC1 mRNA or cDNA occurs, and quantifying the presence of the amplification products. It is anticipated that PCR and/or LCR may be desirable to use as an amplification step in conjunction with any of the techniques used for quantifying nucleic acid detecting.

The methods described herein may be performed, for example, by utilizing prepackaged diagnostic kits comprising at least one probe nucleic acid or set of primer or antibody reagent described herein, which may be conveniently used, e.g., in clinical settings to follow-up or diagnose patients.

The following examples and also the figures and the legends hereinafter have been chosen to provide those skilled in the art with a complete description in order to be able to implement and use the present invention. These examples are not intended to limit the scope of what the inventor considers to be its invention, nor are they intended to show that only the experiments hereinafter were carried out.

### Legends of the Figures

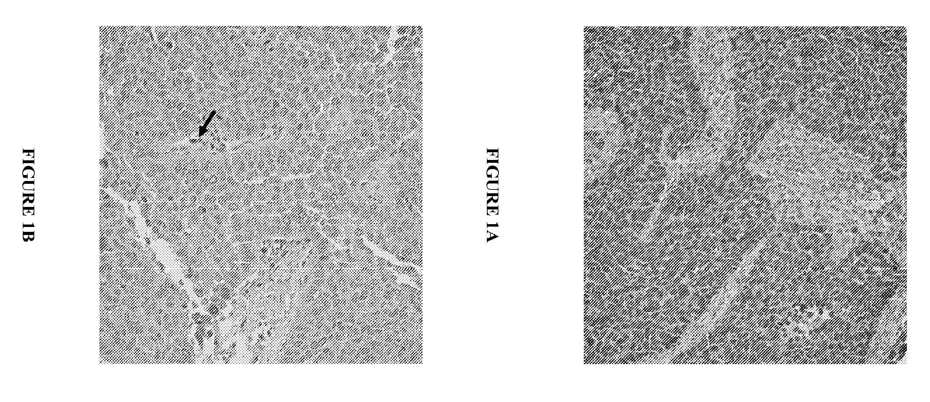

**Figures 1A and 1B****:** Examples of MSH2 positive and MSH2 negative non-small cell lung cancer.
Figure 1A: MSH2 positive case. The staining is intense and diffuse.
Figure 1B: MSH2 negative case. The arrow shows positive stromal cells (internal control).
**Figures 2A-2F****:** Kaplan-Meier estimates of the probability of overall survival

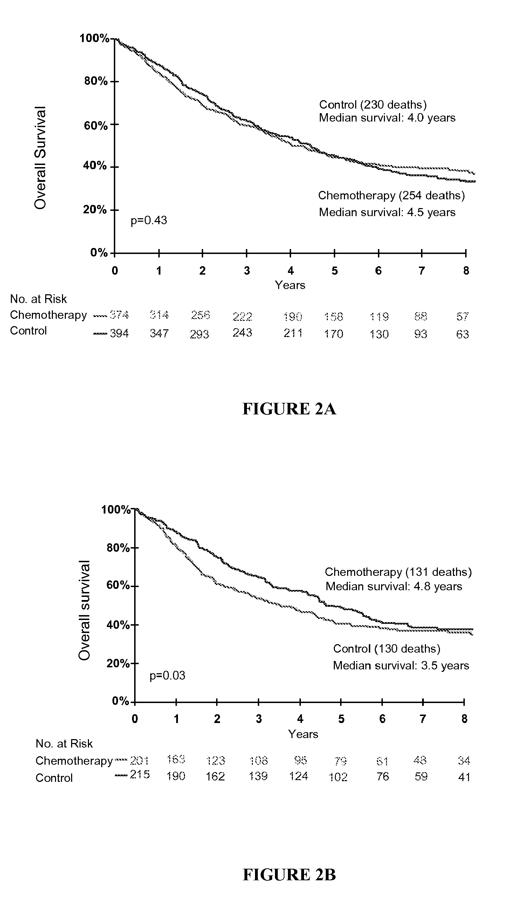

Figure 2A: Patients with MSH2 negative tumours
Figure 2B: Patients with MSH2 positive tumours

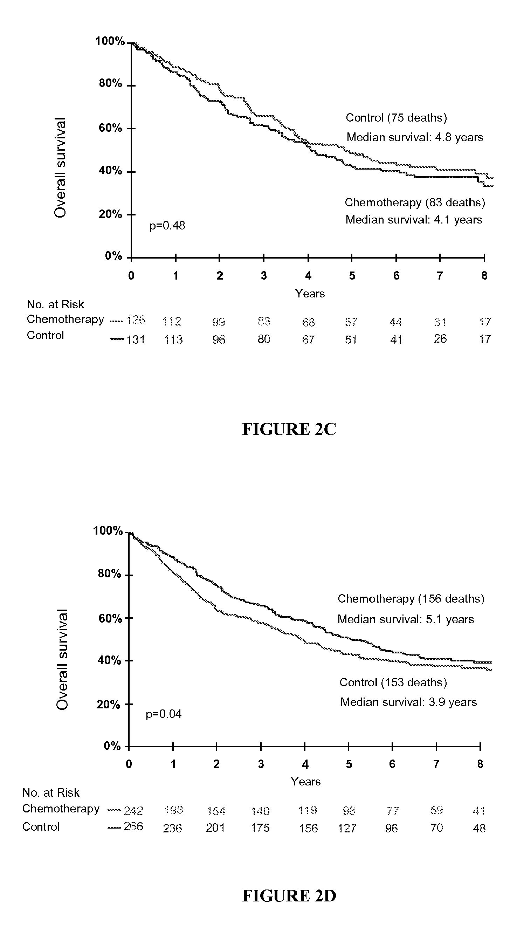

Figure 2C: Patients with MSH2 negative/ERCC1 negative tumours
Figure 2D: Patients with MSH2 positive/ERCC1 positive tumours

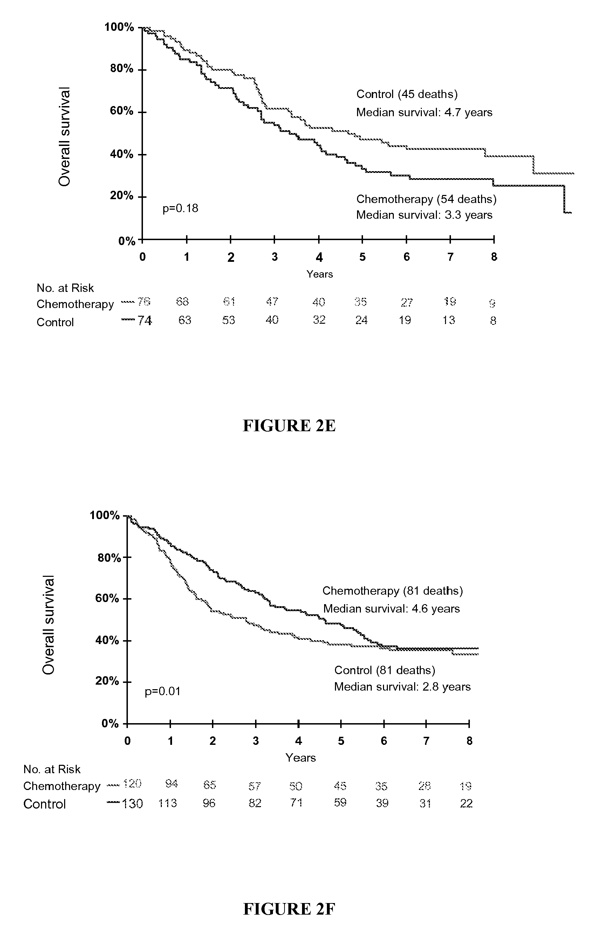

Figure 2E: Patients with MSH2 negative/ERCC1 positive tumours
Figure 2F: Patients with MSH2 positive/ERCC1 negative tumours

### EXAMPLES

### Material and Methods

### A) Patients and study design

All patients had participated in the IALT study (1867 patients) ¹⁷. The IALT-Bio study was designed to examine whether markers predicted survival due to chemotherapy ¹⁸. The 822 patients who were treated in centres that recruited fewer than 10 patients in the clinical IALT study were excluded from the biological study to facilitate specimen collection and to limit the "centre-effect" on the subsequent biomarker analysis. After tissue collection and a pathological review, 783 tissue blocks were included in the IALT-Bio study.

### B) Tissue micro-array (TMA) construction

Three representative tumour areas were selected for each case. Cores measuring 0.6 mm in diameter and 5mm in length (spots) were arrayed following a map. Among the 783 IALT-Bio tissue blocks that contained tumour material, triplicate spots were obtained in 768 (98 %) cases. Random spots were compared to the original blocks to verify agreement between their coordinates and the original samples.

### C) MSH2 immunostaining

Immunostaining was performed following a standard procedure using the Vectastain Elite kit with NovaRED (Vector Laboratories, Burlingame, CA, USA) as the substrate and Mayer's haematoxylin as the counterstain. The primary antibody was the mouse monoclonal antibody FE11 (Calbiochem, San Diego, CA, USA) raised against the C-terminal fragment of human MSH2. For epitope retrieval, slides were heated at 98°C for one hour in 10mM citrate buffer, pH 7.3. Sections were incubated at room temperature for 90 minutes with the FE11 antibody at a dilution of 1:50. The freshly cut TMA sections were manually immunostained in a single experiment that included a tonsil section as an external control.

### D) Evaluation of MSH2 immunostaining

Two investigators who were blinded to clinical data, independently evaluated MSH2 nuclear reactivity. The TMA slides were scanned at high resolution (VM3 virtual scanner, Ziemens, Germany), enabling the study of an identical high-quality image at 20x magnification for each spot for detailed evaluation.

The spots were carefully examined for reactive lung or stromal cells (endothelial cells and fibroblasts), which served as an internal positive control. Spots without a valid internal control were discarded. Cases for which no valid tumour spot could be evaluated were excluded.

Staining intensity was graded on a scale of 0-3, using the expression level in fibroblasts or endothelial cells as a reference (defined *a priori* as a score of 2). The percentage of reactive tumour cells was graded on a scale of 0 %-100 %. A proportion score was assigned to the percentage of reactive tumour nuclei: 0 was assigned if 0 % of reactive tumour nuclei were found, 0.1 if 1 %-9 %, 0.5 if 10 %-49 %, and 1 if ≥50 % of reactive tumour nuclei were present. This proportion score was multiplied by the staining intensity to obtain a histology score (H-score) for each patient¹⁸.

All discordant cases were reviewed in order to reach a consensus.

### E) Statistical analysis

Long-term IALT survival data were used with a median follow-up of 7.5 years 19

A logistic model stratified by centre was used to compare patients with MSH2-positive and MSH2-negative tumours.

The prognostic values of the biomarker status and chemotherapy for overall survival were studied using the Cox model. As in the main IALT analysis ¹⁶, the Cox model included every factor used in the stratified randomisation (centre, tumour stage, and type of surgery) plus clinical and histological prognostic factors (age, sex, WHO performance status, nodal status, lymphoid infiltration, and the revised histopathological type). All other factors that were statistically related to the biomarker status in the multivariate logistic model (P < 0.05) were added to the Cox model.

The predictive value of the biomarker was studied by testing the interaction between the biomarker status and the allocated treatment (chemotherapy versus observation) in the same Cox model. Tests of homogeneity of the hazard ratios were performed within the Cox model. All reported P values were two-sided. In the IALT-Bio analysis plan, P values below 0.01 were considered as statistically significant in order to limit the risk of false positive results. Survival rates were estimated using the Kaplan-Meier method (all P values indicated, besides Kaplan-Meier curves, were adjusted P values corresponding to the Cox analysis).

All analyses were performed using SAS software, version 9.1 (SAS Institute Inc. Cary NC, USA) and curves were drawn with the Tigre^{™} software.

### F) ERRC1 immunostaining

ERCC1-specific immunostaining is carried out as depicted in Olaussen et al. 2006 (18), paragraph " ERCC1 Immunostaining" page 985. The mouse monoclonal "8F1" specifically directed against the full length recombinant human ERCC1 protein being used.

### Example 1: MSH2 expression

Among the 768 patients whose tumour was included in the TMA, no tumour material could be analysed after immunohistochemistry in 34 (4 %) cases. After excluding the cases without valid internal controls, the H-scores were evaluated in 673/768 (88 %) patients.

The H-scores were: 0.2 in 1 case (0 %); 1 in 52 cases (8 %); 2 in 363 cases (54 %); and 3 in 257 cases (38 %). The median H-score was 2 which was chosen to separate positive cases (H-score=3) and negative cases (H-score<3). There were 257 (38 %) positive cases and 416 (62 %) negative cases. Figures 1A-1B show examples of positive and negative cases.

### Example 2: MSH2 expression and baseline clinical characteristics

The relationships between MSH2 expression and the clinical characteristics are provided in Table 1. The proportion of adenocarcinomas was lower (P=0.002) for MSH2-positive cases (20 %) than for MSH2-negative cases (37 %). The morphological slide quality after HE staining was associated with MSH2 expression (P=0.01).

In a logistic model adjusted on sex and slide quality, histology differed according to MSH2 expression, with fewer adenocarcinomas among patients with MSH2-positive tumours (P=0.006).

The 673 cases included in the MSH2 analysis differed from the 95 cases excluded in terms of histology (P<0.001), type of surgery (P=0.02), and slide quality (P=0.01) (there were fewer squamous-cell carcinomas, fewer pneumonectomies, and lower staining quality in excluded cases).

**Table 1. Patient Characteristics***

| **Characteristic** | **Patients with MSH2-Positive Tumours (N=257)** | | **Patients with MSH2 Negative Tumours (N=416)** | | **All Patients (N=673)** | | **P Value†** |
|---|---|---|---|---|---|---|---|
| | **number** | **percent** | **number** | **percent** | **number** | **percent** | |
| **Sex** | | | | | | | 0.05 |
| Male | 221 | 86 | 326 | 78 | 547 | 81 | |
| Female | 36 | 14 | 90 | 22 | 126 | 19 | |
| **Age** | | | | | | | 0.99 |
| <55 years | 70 | 27 | 127 | 31 | 197 | 29 | |
| 55-64 years | 116 | 45 | 181 | 44 | 297 | 44 | |
| >64 years | 71 | 28 | 108 | 26 | 179 | 27 | |
| **Pathological TNM stage** | | | | | | | 0.79 |
| Stage I | 3 | 32 | 150 | 36 | 233 | 35 | |
| Stage II | 53 | 21 | 102 | 25 | 155 | 23 | |
| Stage III | 121 | 47 | 164 | 39 | 285 | 42 | |
| **Tumour** | | | | | | | 0.83 |
| T1 | 36 | 14 | 63 | 15 | 99 | 15 | |
| T2 | 145 | 56 | 263 | 63 | 408 | 61 | |
| T3 | 74 | 29 | 82 | 20 | 156 | 23 | |
| T4 | 2 | 1 | 8 | 2 | 10 | 1 | |
| **Nodes** | | | | | | | 0.99 |
| N0 | 122 | 47 | 188 | 45 | 310 | 46 | |
| N1 | 69 | 27 | 125 | 30 | 194 | 29 | |
| N2 | 66 | 26 | 103 | 25 | 169 | 25 | |
| **Histologic type** | | | | | | | <0.002 |
| Adenocarcinoma | 51 | 20 | 154 | 37 | 205 | 30 | |
| Squamous-cell carcinoma | 174 | 68 | 217 | 52 | 391 | 58 | |
| Other NSCLC | 32 | 12 | 45 | 11 | 77 | 11 | |
| Surgery | | | | | | | 0.34 |
| Pneumonectomy | 118 | 46 | 161 | 39 | 279 | 41 | |
| Lobectomy or segmentectomy | 139 | 54 | 255 | 61 | 394 | 59 | |
| **Performance status score** 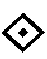 | | | | | | | 0.37 |
| 0 | 147 | 57 | 222 | 53 | 369 | 55 | |
| 1 | 84 | 33 | 165 | 40 | 249 | 37 | |
| 2 | 26 | 10 | 29 | 7 | 55 | 8 | |
| **Lymphoid infiltration** | | | | | | | 0.20 |
| Not intense | 235 | 91 | 360 | 87 | 595 | 88 | |
| Intense | 22 | 9 | 56 | 13 | 78 | 12 | |
| **Pleural invasion** | | | | | | | 0.19 |
| No | 232 | 90 | 386 | 93 | 618 | 92 | |
| Yes | 25 | 10 | 30 | 7 | 55 | 8 | |
| **Vascular invasion** | | | | | | | 0.44 |
| No | 181 | 70 | 295 | 71 | 476 | 71 | |
| Yes | 76 | 30 | 121 | 29 | 197 | 29 | |
| **Lymphatic invasion** | | | | | | | 0.19 |
| No | 71 | 28 | 137 | 33 | 208 | 31 | |
| Yes | 186 | 72 | 279 | 67 | 465 | 69 | |
| **Quality after HE staining** | | | | | | | 0.01 |
| Average | 37 | 14 | 28 | 7 | 65 | 10 | |
| Good | 220 | 86 | 388 | 93 | 608 | 90 | |

| | | | | | | | |
|---|---|---|---|---|---|---|---|
| * TNM denotes tumour-node-metastases. Percentages may not total 100 because of rounding. † P values testing the difference between positive and negative tumours were calculated using logistic regression stratified on centre. 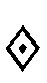 Word Health Organization scores for performance status range from 0 to 2, with a score of 0 indicating no symptoms, 1 mild symptom, and 2 moderate symptoms. | | | | | | | |

### Example 3: Overall survival and adjuvant chemotherapy

For the group of patients included in the MSH2 analysis (673 patients), the adjusted hazard ratio for death associated with chemotherapy compared to observation was 0.88 (95 %CI, 0.72 to 1.07; P=0.21). The 5-year overall survival rates were 47 % (95 %CI, 41 % to 52 %) in the chemotherapy arm, and 44 % (95 %CI, 39 % to 49 %) in the control arm. The 8-year overall survival rates were 36 % (95 %CI, 31 % to 42 %) in the chemotherapy arm, and 37 % (95 %CI, 32 % to 43 %) in the control arm.

### Example 4: Effect of chemotherapy on overall survival according to MSH2 expression

Overall, the effects of adjuvant chemotherapy on the MSH2-negative and positive groups were borderline significantly different (test for interaction, P=0.06). In the MSH2-negative group, overall survival was longer in the chemotherapy arm than in the control arm, (adjusted hazard ratio for death, 0.76; 95 %CI, 0.59 to 0.97; P=0.03) (Table 2, Figure 2A). The 5-year overall survival rates among patients with MSH2-negative tumours were 49% (95%CI, 43% to 56%) in the chemotherapy arm and 41% (95%CI, 34% to 48%) in the control arm. The 8-year overall survival rates among patients with MSH2-negative tumours were 38% (95%CI, 32% to 45%) in the chemotherapy arm and 36% (95%CI, 30% to 43%) in the control arm. Among patients with MHS2-negative tumours, median overall survival was 16 months longer in the chemotherapy arm (58 months) than in the control arm (42 months). In the MSH2-positive group, there was no difference in overall survival between the chemotherapy arm and the control arm (adjusted hazard ratio for death, 1.12; 95%CI, 0.81 to 1.55; P=0.48) (Table 2, Figure 2B). The 5-year overall survival rates among patients with MSH2-positive tumours were 42% (95%CI, 34% to 51%) in the chemotherapy arm and 49% (95%CI, 40% to 58%) in the control arm. The 8-year overall survival rates among patients with MSH2-positive tumours were 34% (95%CI, 25% to 43%) in the chemotherapy arm and 39% (95%CI, 31% to 49%) in the control arm.

**Table 2. Overall survival according to attributed treatment and MSH2 status**

| | **Chemotherapy arm (No deaths / No patients)** | **Control arm (No deaths / No patients)** | **Hazard ratio for death (95% CI)*** | **P value** |
|---|---|---|---|---|
| **Patients with MSH2-negative tumours n = 416** | 131/215 | 130/201 | 0.75 (0.58-0.97) | p=0.03 |
| **Patients with MSH2 positive tumours n = 257** | 83 / 131 | 75/126 | 1.12 (0.81-1.55) | p=048 |
| **Hazard ratio for death (95% CI)**** | 0.99 (0.74-1.32) | 0.66 (0.49-0.90) | - | - |
| **P Value** | p = 0.93 | p = 0.01 | - | p=0.06† |

| | | | | |
|---|---|---|---|---|
| * Hazard ratios are for the comparison of chemotherapy group with the control group. ** Hazard ratios are for the comparison of patients with MSH2-positive tumours with those with MSH2-negative tumours. † The P value is for the interaction between MSH2 expression and treatment. | | | | |

### Example 5: Prognostic effect of MSH2 expression on overall survival

In the control arm, MSH2 positivity compared to MSH2 negativity was associated with an adjusted hazard ratio for death of 0.66 (95 %CI, 0.49 to 0.90; P=0.01). Median overall survival was 16 months longer in the MSH2-positive group (58 months) than in the MSH2-negative group (42 months).

In the chemotherapy arm, there was no difference in overall survival between MSH2-positive and MSH2-negative tumours (adjusted hazard ratio for death, 0.99; 95 %CI, 0.74 to 1.32; P=0.93).

### Example 6: Effect of chemotherapy on overall survival according to MSH2 and ERCC1 expression

The expression levels of MSH2 and ERCC1 were both available for 658 patients (84% of the patients included in IALT-Bio). In a sub-analysis of these 658 patients, the difference between the long-term effects of chemotherapy according to MSH2 or ERCC1 expression were both borderline significant (test for interaction, P=0.05 and P=0.04, respectively).

In the MSH2-negative group (404 patients), the adjusted hazard ratio for death associated with chemotherapy versus observation was 0.75 (95 %CI, 0.58 to 0.98; P=0.03)(Table 3). In the MSH2-positive group (254 patients), there was no difference in overall survival between the chemotherapy arm and the control arm (adjusted hazard ratio for death, 1.14; 95 %CI, 0.82 to 1.57; P=0.44).

Among these 658 patients, the adjusted hazard ratio for death associated with chemotherapy versus observation was 0.73 (95 %CI, 0.55 to 0.96; P=0.03) in the ERCC1-negative group (354 patients) (Table 3). In the ERCC1-positive group (304 patients), there was no difference in overall survival between the chemotherapy arm and the control arm (adjusted hazard ratio for death, 1.11; 95 %CI, 0.83 to 1.50; P=0.49).

### Example 7: Relationships between MSH2 and ERCC1 expression

The proportion of MSH2-positive cases was 29 % (104 of 354 cases) in the ERCC1-negative group and 49 % (150 of 304 cases) in the ERCC1-positive group (p<0.001). The association of MSH2 with ERCC1 remained in a model adjusted on slide quality and histological type (p<0.001).

### Example 8: Predictive value of combining MSH2 and ERCC1

The effect of chemotherapy was examined in the following subgroups defined by combining MSH2 and ERCC1: MSH2-positive/ERCC1-positive, MSH2-positive/ERCC1-negative, MSH2-negative/ERCC1-positive, MSH2-negative/ERCC1-negative (Figures 2C to 2F). The differential effect of chemotherapy for patients with MSH2-positive tumours compared to those with MSH2-negative lesions was similar among patients with ERCC1-positive and ERCC1-negative tumours (P=0.94), i.e., the ratio of the hazard ratios associated with MSH2 among patients with ERCC1-negative (0.90/0.65) was equal to that among patients with ERCC1-positive tumours (1.32/0.92) (Table 3).

**Table 3. Overall survival according to allocated treatment and subgroup analysis**

| | **Patients with MSH2-Negative tumours** | **Patients with MSH2-Positive tumours** | **Total** |
|---|---|---|---|
| **Patients with ERCC1-negative tumours** Number of death / number of patients | 162 / 250 | 57 / 104 | |
| Hazard ratio for death (95% CI) P Value | 0.65 (0.47-0.91)* p=0.01 | 0.90 (0.52-1.55)* p=0.70 | 0.73 (0.55-0.96)* |
| **Patients with ERCC1-positive tumours** Number of death / number of patients | 90 / 154 | 99/150 | |
| Hazard ratio for death (95% CI) P Value | 0.92 (0.60-1.42)* P=0.71 | 1.32 (0.88-1.99)* P=0.19 | 1.11 (0.83-1.50)* |
| **Total** Hazard ratio for death (95% CI) | 0.75 (0.58-0.98)* | 1.14 (0.82-1.57)* | - |
| | P=0.03 | P=0.44 | |

| | | | |
|---|---|---|---|
| * Hazard ratios are for the comparison of the chemotherapy group with the control group. | | | |

In the combined MSH2-negative/ERCC1-negative subgroup, overall survival was longer in the chemotherapy arm than in the control arm (adjusted hazard ratio for death, 0.65; 95 %CI, 0.47 to 0.91; P=0.01) (Figure 2C). In the MSH2-positive/ERCC1-positive subgroup, the hazard ratio for death associated with chemotherapy was 1.32 (95%CI, 0.88 to 1.99; P=0.19) (Figure 2D). The adjusted hazard ratio for death associated with chemotherapy when 1 marker was positive (either MSH2 or ERCC1 but not both) was 0.91 (95 %CI, 0.65 to 1.28). When we defined a score by counting the number of positive MSH2 and ERCC1 markers in the above-defined subgroups (allocating a score of 0 for no positive marker, 1 for 1 positive marker and 2 for 2 positive markers), the test of a decreasing effect of chemotherapy with an increasing score was significant (P=0.01).

The results demonstrate that the long-term survival benefit derived from adjuvant chemotherapy may be different according to MSH2 expression in cancer patient, particularly in NSCLC. Patients in the MSH2-positive group did not benefit from chemotherapy, while patients in the MSH2-negative group did. In the MSH2-negative group, there was a reduced adjusted hazard ratio for death of 25 %, and a gain in median survival of 16 months in favour of chemotherapy versus observation. When the analysis focused exclusively on the control arm, MSH2 positivity was associated with longer overall survival. The results are strengthened by the adjustments for multivariate overall survival predictors and by the use of two-sided statistical tests. They demonstrate that MSH2 is prognostic in patients under observation and is a predictor of overall survival benefit from cisplatin-based chemotherapy.

A previous study already demonstrated a survival benefit from adjuvant cisplatin-based chemotherapy in patients whose tumours were ERCC1 negative which was not the case for patients with ERCC1-positive lesions ¹⁸. Furthermore, the prognostic role of ERCC1 tumour expression suggested by our data was later strongly supported by Zheng et al ²⁰. In the present study, we used recently updated survival data from the IALT study (7.5 years of median survival) which for the first time allowed us to evaluate DNA repair markers as predictors of the long-term benefit of cisplatin-based chemotherapy ¹⁹. The long-term predictive value of MSH2 was equal to that of ERCC1 in patients for whom both markers were available.

Both MSH2 and ERCC1 are involved in the repair of cisplatin-induced DNA lesions ^{5, 6}. Here, the inventors have demonstrated that they are co-ordinately expressed. The differential effect of chemotherapy on patients with MSH2-positive and MSH2-negative tumours was similar among patients with ERCC1-positive and ERCC1-negative tumours, suggesting that the predictive value of MSH2 is partly independent of that of ERCC1. Indeed, when both markers were negative, the long-term effect of chemotherapy was associated with a reduced adjusted hazard ratio for death of 35 %, which highly suggests that the predictive value of MSH2 and that of ERCC1 act cumulatively. Whereas the data do not allow us to infer that chemotherapy was detrimental when both markers were positive, this possibility cannot be ruled out.

Loss of MSH2 is associated with mismatch repair deficiency and the resulting microsatellite instability in colon and endometrial cancers, but not in lung cancer ^{21, 22}. This suggests that mismatch repair deficiency is not responsible for the effects of the loss of MSH2 on cisplatin resistance in NSCLC.

In conclusion, the results of the present study indicate that MSH2 is prognostic in patients under observation and predictive of long-term overall survival benefit from platinum-based chemotherapy, particularly for cisplatin-based chemotherapy. These results also demonstrate the potential use of MSH2 together with ERCC1 in the clinical setting.

### REFERENCES

1. Gazdar AF. DNA repair and survival in lung cancer--the two faces of Janus. The New England journal of medicine 2007;356(8):771-3.
2. Fishel R, Lescoe MK, Rao MR, et al. The human mutator gene homolog MSH2 and its association with hereditary nonpolyposis colon cancer. Cell 1993;75(5):1027-38.
3. Duckett DR, Drummond JT, Murchie AI, et al. Human MutSalpha recognizes damaged DNA base pairs containing O6-methylguanine, O4-methylthymine, or the cisplatin-d(GpG) adduct. Proc Natl Acad Sci U S A 1996;93(13):6443-7.
4. Zdraveski ZZ, Mello JA, Farinelli CK, Essigmann JM, Marinus MG. MutS preferentially recognizes cisplatin- over oxaliplatin-modified DNA. J Biol Chem 2002;277(2):1255-60.
5. Lan L, Hayashi T, Rabeya RM, et al. Functional and physical interactions between ERCC1 and MSH2 complexes for resistance to cis-diamminedichloroplatinum(II) in mammalian cells. DNA Repair (Amst) 2004;3(2):135-43.
6. Zhang N, Liu X, Li L, Legerski R. Double-strand breaks induce homologous recombinational repair of interstrand cross-links via cooperation of MSH2, ERCC1-XPF, REV3, and the Fanconi anemia pathway. DNA Repair (Amst) 2007;6(11):1670-8.
7. Xinarianos G, Liloglou T, Prime W, et al. hMLH1 and hMSH2 expression correlates with allelic imbalance on chromosome 3p in non-small cell lung carcinomas. Cancer Res 2000;60(15):4216-21.
8. Brooks KR, To K, Joshi MB, et al. Measurement of chemoresistance markers in patients with stage III non-small cell lung cancer: a novel approach for patient selection. Ann Thorac Surg 2003;76(1):187-93; discussion 93.
9. Wang YC, Lu YP, Tseng RC, et al. Inactivation of hMLH1 and hMSH2 by promoter methylation in primary non-small cell lung tumors and matched sputum samples. J Clin Invest 2003;111(6):887-95.
10. Zhu LQ, Diao LM, Chen DJ, Li HG, Liu X, Zou ZY, Li BY, Wang M, Liu MQ. Relationship between hMSH2 and FHIT gene expression in non-small cell lung cancer Ai Zheng. 2003;22(6):571-4.
11. Hsu HS, Wen CK, Tang YA, et al. Promoter hypermethylation is the predominant mechanism in hMLH1 and hMSH2 deregulation and is a poor prognostic factor in nonsmoking lung cancer. Clin Cancer Res 2005;11(15):5410-6.
12. Skarda J, Fridman E, Plevova P, et al. Prognostic value of hMLH1 and hMSH2 immunohistochemical expression in non-small cell lung cancer. A tissue microarray study. Biomed Pap Med Fac Univ Palacky Olomouc Czech Repub 2006;150(2):255-9.
13. Kanellis G, Chatzistamou I, Koutselini H, et al. Expression of DNA mismatch repair gene MSH2 in cytological material from lung cancer patients. Diagn Cytopathol 2006;34(7):463-6.
14. Scartozzi M, Franciosi V, Campanini N, et al. Mismatch repair system (MMR) status correlates with response and survival in non-small cell lung cancer (NSCLC) patients. Lung Cancer 2006;53(1):103-9.
15. Hsu HS, Lee IH, Hsu WH, Kao WT, Wang YC. Polymorphism in the hMSH2 gene (gISV12-6T > C) is a prognostic factor in non-small cell lung cancer. Lung Cancer 2007;58(1):123-30.
16. Cooper WA, Kohonen-Corish MR, Chan C, et al. Prognostic significance of DNA repair proteins MLH1, MSH2 and MGMT expression in non-small-cell lung cancer and precursor lesions. Histopathology 2008;52(5):613-22.
17. Arriagada R, Bergman B, Dunant A, Le Chevalier T, Pignon JP, Vansteenkiste J. Cisplatin-based adjuvant chemotherapy in patients with completely resected non-small-cell lung cancer. The New England journal of medicine 2004;350(4):351-60.
18. Olaussen KA, Dunant A, Fouret P, et al. DNA repair by ERCC1 in non-small-cell lung cancer and cisplatin-based adjuvant chemotherapy. The New England journal of medicine 2006;355(10):983-91.
19. Le Chevalier T, Dunant A, Arriagada R, et al. Long-term results of the International Adjuvant Lung Cancer Trial (IALT) evaluating adjuvant cisplatin-based chemotherapy in non-small cell lung cancer (NSCLC). J Clin Oncol 2008;26(15 S Suppl): 398s.
20. Zheng Z, Chen T, Li X, Haura E, Sharma A, Bepler G. DNA synthesis and repair genes RRM1 and ERCC1 in lung cancer. The New England journal of medicine 2007;356(8):800-8.
21. Forgacs E, Wren JD, Kamibayashi C, et al. Searching for microsatellite mutations in coding regions in lung, breast, ovarian and colorectal cancers. Oncogene 2001;20(8):1005-9.
22. Weinberg RA. The biology of cancer. Garland Science Eds 2007:463-556. ly, the ERCC1 expression level.

## Claims

1. A method for *in vitro* predicting the benefit of the response of a subject diagnosed with cancer to a platinum-based chemotherapy from a biological sample from said subject comprising the following steps of:
a) determining the MSH2 expression level in the biological sample; and
b) optionally, comparing said MSH2 expression level to the MSH2 expression level of a reference control or population, or compared to the non tumor cells content.

2. The method claim 2, further comprising
c) determining the ERCC1 expression level from the same or from another biological sample; and
d) optionally, comparing said ERCC1 expression level to the ERCC1 expression level of a reference control or population, or compared to the non tumor cells content.

3. The method according to claim 1, wherein in step b), if the MSH2 expression level obtained for the patient biological sample is less or equal than the expression level obtained for said reference control or population, or is decreased compared to the non tumor cells content, then an overall survival benefit can be predicted.

4. The method according to claim 2, wherein in step b) and in step d), if the MSH2 and the ERCC1 expression levels obtained for the patient biological sample are less or equal than the expression level obtained for said reference population, or is decreased compared to the non tumor cells content, then an overall survival benefit can be predicted.

5. A method for in *vitro* assessing whether a platinum-based chemotherapy is appropriate for a subject diagnosed with cancer from a biological sample from said subject, said method comprising the following steps of:
a) determining the MSH2, and optionally the ERCC1, expression level in said biological sample; and
b) optionally, comparing said MSH2, and optionally ERCC1, expression level to the MSH2, and optionally ERCC1, of a reference control or population, or compared to the non tumor cells content
- a platinum-based chemotherapy will be determined as an appropriate chemotherapy if the MSH2, and optionally the ERCC1, expression level is less or equal than the MSH2, and optionally the ERCC1, expression level of a reference control or population, or is decreased compared to the non tumor cells, and
- a platinum-free chemotherapy will be determined as an appropriate chemotherapy if the MSH2, and optionally the ERCC1, expression level is greater than the MSH2, and optionally the ERCC1, expression level of said reference control or population, or is increased compared to the non tumor cells.

6. An *in vitro* screening method for selecting a subject diagnosed from cancer for a treatment with a platinum-based chemotherapy from a biological sample from said subject, said method comprising the steps of:
a) determining the MSH2, and optionally the ERCC1, expression level in said biological sample; and
b) comparing said MSH2, and optionally ERCC1, expression level to the MSH2, and optionally ERCC1, of a reference control or population, or compared to the non tumor cells content,
wherein said subject will be selected for a platinum-based chemotherapy if the MSH2, and optionally the ERCC1, expression level is less or equal than the MSH2, and optionally the ERCC1, expression level of a reference control or population, or is decreased compared to the non tumor cells content.

7. The method of ant one of claims 1 to 6, wherein said subject is a human patient.

8. The method of any one of claims 1 to 7, wherein said chemotherapy is an adjuvant-platinum-based chemotherapy.

9. The method of any one of claims 1 to 8, wherein said platinum-based chemotherapy is cisplatin-based chemotherapy.

10. MSH2 as a prognostic marker associated with longer overall survival of a subject suffering from cancer.

11. The method of any one of claims 1-9 and the marker of claim 10, wherein the subject has a malignant mesothelioma, a bladder cancer, a testicular cancer, cancer of the upper aero-digestive tract or ovarian cancer.

12. The method of any one of claims 1 to 9 and the marker of claim 10, wherein the subject has non- small-cell lung cancer.

13. The method of any one of claims 1 to 12, wherein said biological sample is a tissue sample comprising cancer cells, particularly a biopsy containing tumor cells.

14. The method of any one of claims 1 to 13, wherein in step a), said MSH2 expression and/or said ERCC1 expression level(s) to be determined is (are) the level of the RNA transcript or their expression protein product of the MSH2 and/or ERCC1 gene.

15. The method of claim 14 wherein the MSH2 and/or ERCC1 expression level is determined by:
- a method including a PCR or a RT-PCR method, or a Northern method when the determined MSH2 and/or ERCC1 expression product is the RNA transcript; or
- a Western blot method or an immunohistochemistry method when the determined MSH2 and/or ERCC1 expression product is the MSH2 and/or ERCC1 protein, or a specific fragment thereof.

16. Kit or array wherein said kit or array comprises a reagent for assaying MSH2 expression and a reagent for assaying ERCC1 expression in a biological sample from a patient.

17. The kit or array of claim 16, wherein the reagent for assaying MSH2 and the reagent for assaying ERCC1 expression comprises a reagent selected from the group consisting of:
- a probe or a pair of primers that specifically hybridizes to the MSH2 or to the ERCC1 mRNA or cDNA; or
- an anti-MSH2 capable of specifically recognizing the MSH2 protein or an anti-ERCC1 antibody capable of specifically recognizing the ERCC1 protein.
